# EUROPEAN PATENT SPECIFICATION

(11) **EP 0 861 117 B1**
(45) Date of publication and mention of the grant of the patent: **25.07.2001**
(21) Application number: 96939409.7
(22) Date of filing: 12.11.1996
(51) Int. Cl.: B01F 3/12, B01F 13/06, B01F 15/02, A61F 2/46

(54) **METHOD AND DEVICE FOR MIXING COMPONENTS FOR BONE CEMENT IN A MIXING VESSEL**
VERFAHREN UND VORRICHTUNG ZUM MISCHEN VON BESTANDTEILEN FÜR KNOCHENZEMENT IN EINEM MISCHBEHÄLTER
PROCEDE ET DISPOSITIF POUR MELANGER DES COMPOSANTS D'UN CIMENT OSSEUX DANS UNE VASE DE MELANGE

(30) Priority: 13.11.1995 US 545591; 22.10.1996 US 734817
(43) Date of publication of application: 02.09.1998
(60) Divisional application: 00101313.5
(73) Proprietor: CEMVAC SYSTEM AKTIEBOLAG, 589 41 Linköping (SE)
(72) Inventor: JONSSON, Sören, S-589 41 Linköping (SE)
(74) Representative: Willquist, Bo
(86) International application number: SE9601459
(87) International publication number: WO9718031

(56) References cited:
- WO-A-93/22041
- WO-A-94/26403

## Description

### BACKGROUND OF THE INVENTION

### Field of the Invention

The present invention relates to a method for feeding of constituent components in a mixing vessel for the preparation of bone cement under vacuum. The invention also relates to an apparatus for feeding of constituent components in a mixing vessel under partial vacuum for the preparation of bone cement.

### Description of the Prior Art

Bone cement is prepared by mixing polymethyl methacrylate in powder form, with liquid monomethyl methacrylate in a mixing container. Both the liquid component and the combined mixture give off substances in gaseous form which are environmentally harmful and injurious to the health. For this reason, it is important for the introduction of the bone cement components into the mixing container and the mixing process itself, to take place in such a way that the smallest possible quantity of the harmful gases escape into the surrounding environment Mixing vessels in which the introduced components were successfully prepared into bone cement without a substantial release of the aforementioned gases are described in SE-C-8901599-4, SE-A0-9201353-1 and WO 94/26403, for example. In order for the bone cement to develop optimal strength during use, it is also important for the components comprising the cement to have well-mixed, predetermined portions.

In said WO-publication a glass ampoule containing a liquid component of a bone cement is siurounded by a container which is in reclosable communication with the atmosphere. A second container surrounds the first container so that when the mixing vessel is opened, the contents of the ampoule, under the effect of a partial vacuum inside the mixing vessel, can be sucked down into it, a space formed between the aforementioned inner container and outer container is filled with a second bone cement component in powder form which is caused by displacement of the inner container relative to the outer container, to move from a first position in which the space does not communicate with the atmosphere or the mixing vessel to a second position in which the space communicates with the atmosphere and the mixing vessel, so that the powdered bone cement component is sucked down into the mixing vessel under the effect of the partial vacuum inside it.

A device for carrying out the above method which device is also explained in said WO-publication is characterized in that it comprises, an inner container communicating with the atmosphere, and is so arranged as to enclose the glass ampoule containing the liquid bone cement component, and to communicate with the aforementioned mixing vessel, and which includes means for opening the ampoule so that its contents, under the effect of the partial vacuum inside the mixing vessel, can be sucked down into it. The outer container at least partially encloses the inner container and is also arranged so as to communicate with the mixing vessel and together with the inner container, defines a space therebetween which is filled with a certain quantity of the powdered component of bone cement. The inner container is capable of displacement from a first position to a second position, the first position characterized by the inner container preventing communication between both the mixing vessel and the atmosphere, and the second position characterized, in which communication between the mixing vessel on the one hand and the atmosphere on the other hand is open, so that the bone cement component in powder form, under the effect of the partial vacuum inside the mixing vessel, can be sucked into it without escape of gases.

### SUMMARY OF THE INVENTION

One object of the present invention is to make available a method and device of the kind described above, which avoids the risk of gas release when feeding the bone cement components into the mixing vessel. Another object is to make the mixing as convenient and simple as possible for a person using the device. Still another object is to make the mixing as safe as possible for said person i.e. reduce to a minimum the failure rate of mixing. This is achieved in accordance with the invention in a number of ways. According to one method, which has a couple of variants, the mixing vessel is prefilled with the powder bone cement component. The first container holding the ampoule of the liquid bone cement component connects to the mixing vessel in a manner where displacement of a container cap causes the ampoule to break and the liquid component to be sucked into the mixing vessel under vacuum.

According to another method, the liquid bone cement component is supplied through a collapsible plastic bag and tubing arrangement attached thereto. The tubing can be connected to the mixing vessel in at least two convenient locations, where a tubing clamp is released to allow the liquid component to flow into the mixing vessel under the influence of vacuum.

Another method of the present invention simply involves providing a glass ampoule with the liquid component therein, breaking the ampoule, and then supplying the contents into the mixing vessel through a funnel attached thereto. Again the liquid component is sucked under vacuum into the mixing vessel.

The devices for carrying out all methods in accordance with this invention are more fully detailed in the following sections.

### BRIEF DESCRIPTION OF THE DRAWINGS

The invention is described below in greater detail with reference to the accompanying drawing, in which:
Figure 1 illustrates the mixing vessel of a first embodiment of the present invention in which the powder form of the bone cement pre-exists within the mixing vessel;
Figure 2 illustrates a container and a cassette that houses a glass ampoule which contains the liquid form of the bone cement;
Figure 3 illustrates a cross-sectional view of the mixing vessel of Figure 1 prior to feeding the liquid component into the vessel prefilled with powder;
Figure 4 illustrates the mixing vessel of figure 3, feeding the liquid into the vessel;
Figure 5 illustrates the mixing vessel of Figure 1 with the bone cement components being thoroughly mixed;
Figure 6 illustrates a second embodiment of the present invention, where the liquid bone cement component is contained in a plastic bag;
Figure 7 illustrates the mixing vessel of an third embodiment of the present invention where the liquid form of the bone cement component is contained in a separate container and feed through a funnel to admix with the powder bone cement component.

### DETAILED DESCRIPTION OF THE INVENTION

The designations 1 and 2 are used generally in the drawing in respect of a feed arrangement and a mixing vessel. The latter comprises an interior 2a, a cylindrical container 3 comprised of an outer cylinder wall 3a, a bottom 4 at one end of the container and a spout 5 with sealed opening at the other end, together with an agitator 6, received within said spout and mixing vessel and capable of axial, vertical movement inside the container 3. The agitator 6 consists of an agitator disc 6a attached to a tubular agitator rod 6b. The agitator 6 is mounted so that it is free to vertically slide up and down while maintaining a seal in the spout 5, in such a way that the plurality of holes 6h in agitator disk 6a can be used to bring about through mixing of the bone cement components A, B within mixing vessel 2 with no gaseous escape to atmosphere. Once mixing is complete, and once a lock 7 has been removed, the bottom 4 can be axially displaced inside the cylinder by upward movement of piston head 80, moving towards the spout 5. The piston-like function of bottom 4, upwardly pushes and then discharges the mixed bone cement via the hollow agitator rod 6b, which now serves as a discharge nozzle. The interior of the container 2 communicates via a first filter 8 and a second filter 21 with a vacuum source (not shown) during feeding and mixing of the bone cement components A, B. Rapid and effective feeding of the bone cement components A, B into the mixing vessel, and safe handling of the gases that are environmentally harmful and injurious to human health, are achieved in this way.

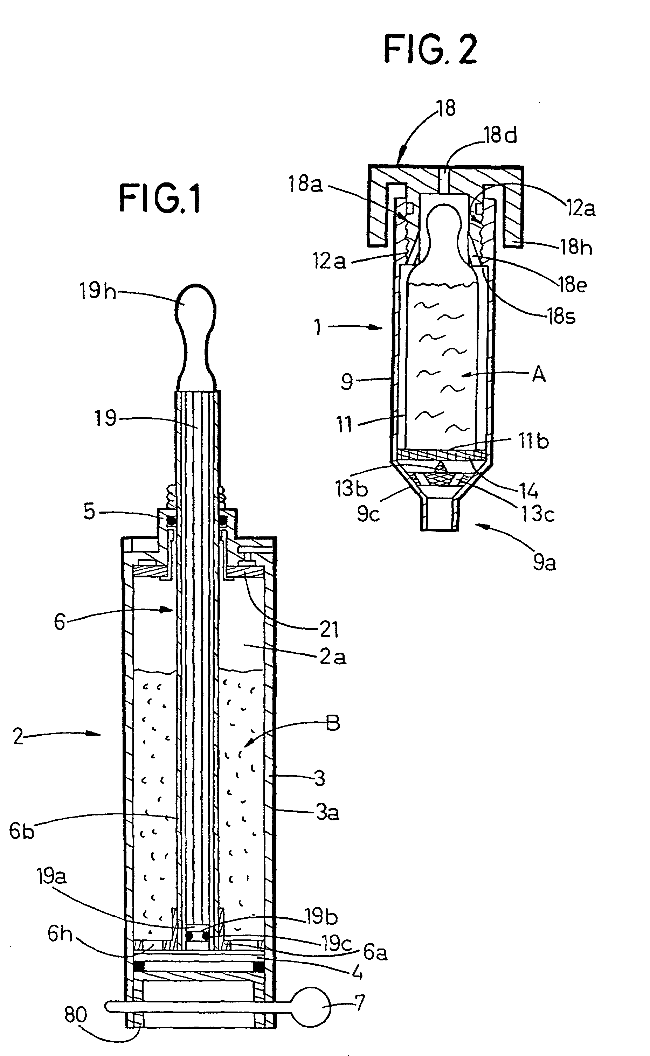

Turning attention now to Figures 1-6, a first and a second embodiment of the present invention will now be described. The powdered component B of bone cement pre-exists within the mixing vessel 2 prior to any mixing procedures, and the container 9 contains only the liquid component A. It will become clearer after reading the following description that the main characteristic of the first embodiment is that the liquid component A will be drawn into the mixing vessel under vacuum, and that an ampoule arrangement is provided wherein the ampoule rests on the mixing vessel and wherein the contents feed downward through the tubular agitator rod 6b, and enter vessel 2 in the vicinity of the vessel bottom 4.

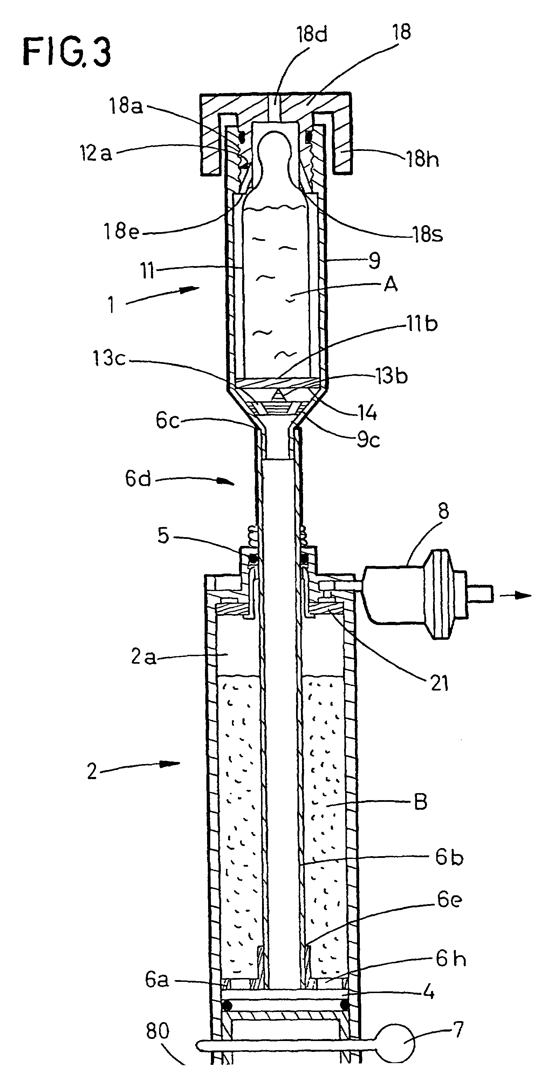

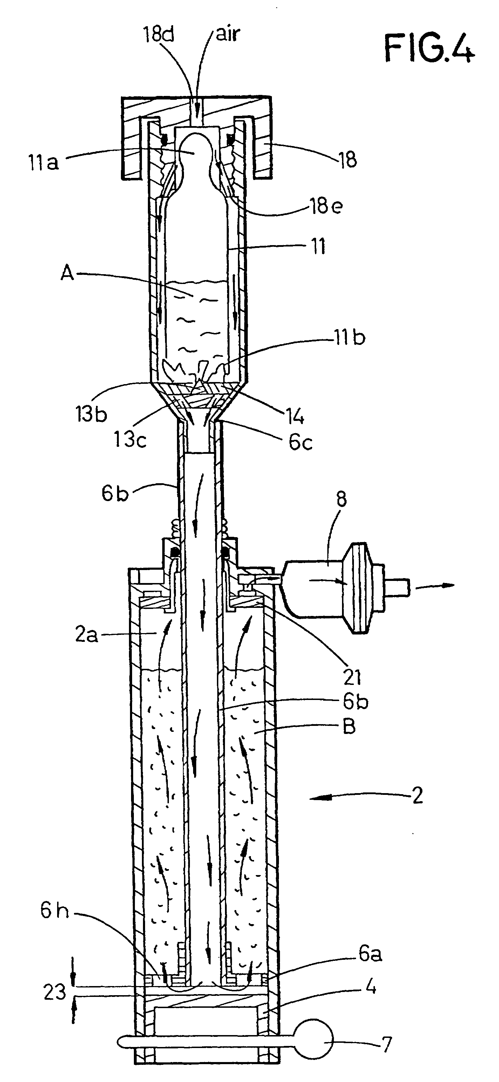

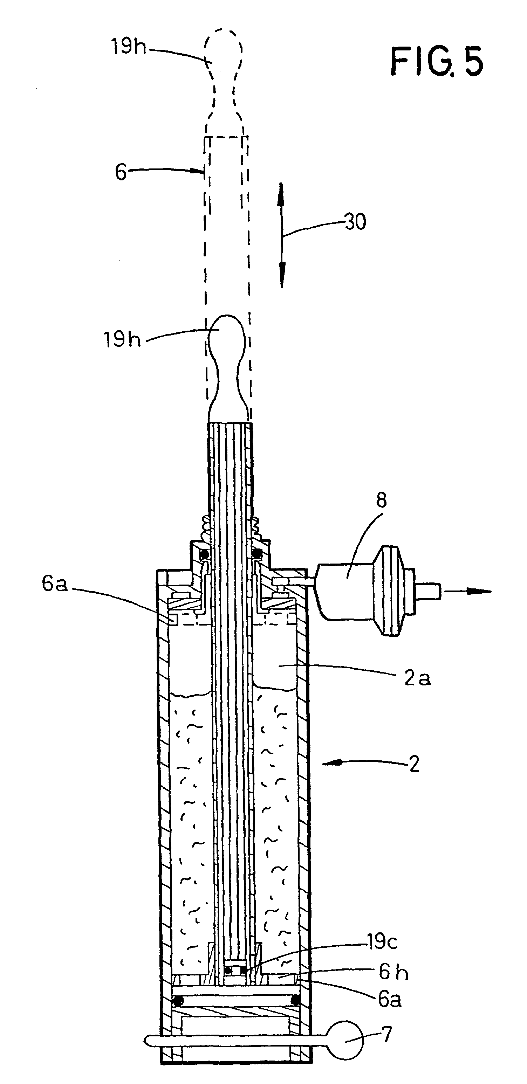

In accordance with the first embodiment, Figures 1-5 show mixing vessel 2 as being prefilled with the powder component B of the bone cement. A tightening rod 19 is received within tubular agitator rod 6b of agitator 6 and has plug 19a and O-ring 19c inserted within a groove 19b thereof, to form an air-tight seal so that powdered contents B are not contacted by and affected by atmospheric air which is capable of downwardly travelling along tubular rod 6b to vessel bottom 4. Just prior to introducing glass ampoule 11 on top of mixing vessel 2, tightening rod 19 is completely removed from tube 6b, wherein the cylindrical container 9 is placed on top of vessel 2 by inserting funnel-shaped neck 9c into mouth 6c at the first end 6d of tubular rod 6b. Figure 2 shows in greater detail that glass ampoule tip 11a is pointing upwards when inserted within container 9, and that the ampoule is resting upon the upward cone 13b of breaking means 13, said means having internal passages 13c for allowing liquid there through once it passes filter 14. Figure 2 also illustrates that cap 18 has gripping means 18h for facilitating the operative threading movement of cap 18 along container threads 12a. Figure 3 shows the ampoule 11 just prior to being broken. Figure 4 shows that when handle 18h of the cap is turned so as to downwardly displace the cap 18 through action of the interacting threads 12a and 18a, shoulder 18s pushed downwardly against ampoule 11, causing upward-facing, pointed cone 13b to break bottom 11b of the ampoule, thereby allowing liquid contents A to flow through filter 14 under suction downwardly into hollow tube 6b as previously described. As Figure 4 shows, openings 18d and 18e in cap 18, allow atmospheric air to be communicated into the interior of container 9 under suction, also as previously described, thereby preventing noxious fumes escaping to atmosphere. A small gap 23 exists between vessel bottom 4 and agitator disk 6a so that as liquid A descends tubular rod 6b, exists open end 6e, then it enters gap 23, which behaves as a passage for percolating an air/liquid mixture upwardly through holes 6h in the agitator disk 6a, so that air bubbles cause liquid A to thoroughly mix with the powder component B, while under the continuing action of the vacuum source. Figure 5 illustrates that once ampoule 11 is empty, container 9 is removed and replaced with tightening rod 19. While still under vacuum, tubular rod 6b is grasped and then successively moved up and down in the direction of arrow 30 and down with rod 19 still inserted therein, as the outlined representation in Figure 5, so that agitator disk 6a ensures thorough mixing of the liquid and powder components A, B, while rod 19 prevents gaseous escape from tubular rod 6b due to O-ring seal 19c. The filter 21 is provided to prevent heavy particulate from being drawn into the vacuum source not shown. Once admixed, first lock 7 and then rod 19 are removed and bottom 4 can be axially displaced within cylinder 3 in a fashion similar to a piston, as previously described, so that the mixed bone cement can be pushed out of vessel 2 once tightening rod 19 is removed. In this way, agitator rod 6 is pulled completely up so that agitator disk 6a contacts the top end 3b of cylinder 3, with tubular rod 6b acting as a discharge nozzle for the now-ready cement.

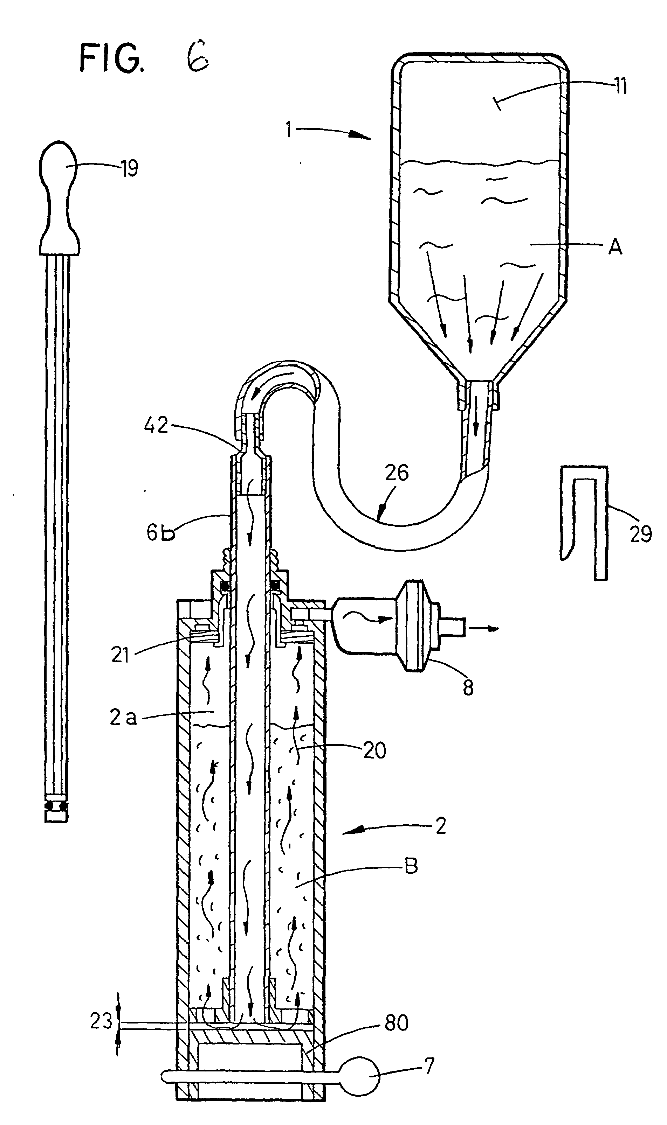

Figure 6 shows a second embodiment of the present invention where it is seen that the means for introducing the liquid into the mixing vessel is now in the form of a collapsible bag of which is directly inserted into tubular agitator 6b once tightening rod 19 is removed, as is shown. Then, the U-shaped sleeve member 29 which has been used as a valve, where tube 26 is folded and frictionally inserted within sleeve interior 29a, is removed from tube 26 so that liuqid component A is drawn under the influence of vacuum down to the bottom of tubular agitator rod 6b, and be mixed as previously described. An airtight adapter means 42 is provided at the tube end so as to securely hold it within tube 26 during introduction of the liquid A.

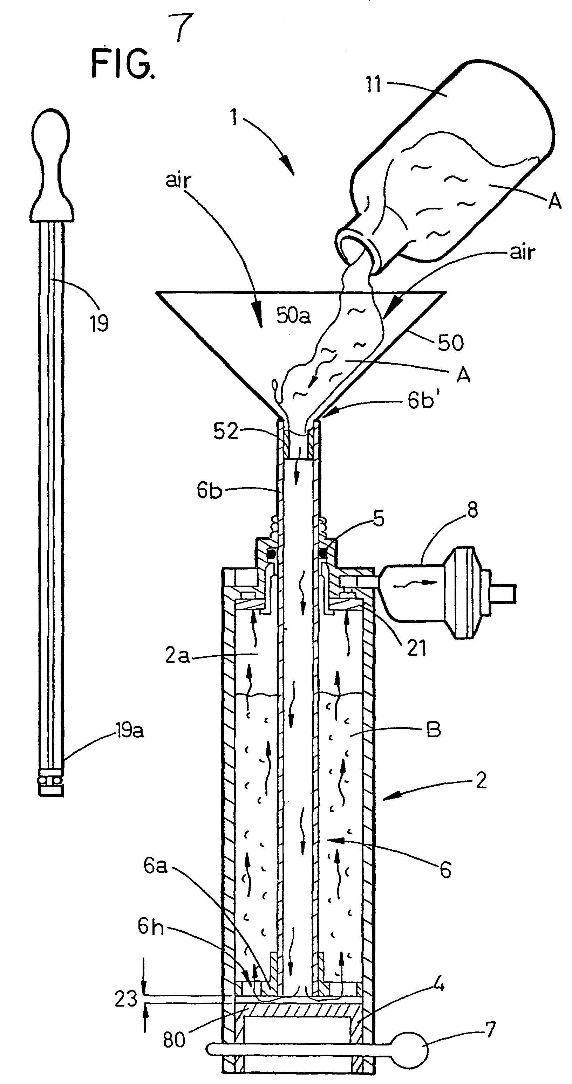

Figure 7 shows a third embodiment of the present invention where it is seen that the cylindrical containers 9 and 10 are eliminated so that once the glass ampoule 11 is broken, the liquid bone cement component A, is introduced into mixing vessel 2 via removable funnel member 50 and hollow agitator rod 6b. As seen open funnel neck 52 is in frictional engagement with the open mouth 6b' of rod 6b when funnel 50 is inserted therein. The tip 11a of ampoule 11 is broken off and contents A are fed into funnel component receiving area 50a before descending down tube 6b. Again, this arrangement provides the liquid component A at the container bottom so that both components A, B can be pre-mixed together through percolation as previously explained. During filling of component A prior to mixing, the suction pressure on vacuum causes high velocity of ambient air entering into the funnel 50 which will prevent fumes from escaping from mixing vessel 2 out into the operating theatre. It is envisioned that funnel member 50 be provided with an O-ring or similar seal about its open neck 52.

## Claims

1. A system for mixing a liquid and a powder component (A, B) for the preparation of a bone cement under vacuum in order to prevent harmful emissions from escaping once said liquid and powder components (A, B) are mixed, comprising:
a mixing vessel (2) defined by an outer wall (3) having a top end, a bottom end and an interior, said top end formed with a sealable spout (5), said bottom end formed with an axially displaceable bottom (4);
a means (1) for introducing by aid of vacuum present in the vessel said liquid component (A) into said interior of said mixing vessel (2) through said sealable spout (5);
an agitator (6) received within said vessel interior (2a), said agitator (6) being mounted so that it is free to vertically slide up and down for mixing components (A) and (B) while maintaining a seal in the spout (5) and of a tubular rod (6b) having an open upper end defining a mouth extending upwardly out of said interior through said spout (5) and in communication with the atmosphere and an agitator disk (6a) attached to said tubular rod (6b), at an open, bottow end of said tubular rod within the vessel interior;
a removable tightening rod (19) disposed within said tubular rod (6b) for sealing said open bottom rod end from communication with the atmosphere prior and after said liquid component (A) is introduced into said mixing vessel (2), said tightening rod (19) being removed from said tubular rod (6b) immediately prior to said liquid bone cement (A) compound is introduced into said mixing vessel (2) and being reinserted therein after said liquid (A) has being introduced within said mixing vessel, **characterised** in that said vessel being pre-filled with said powder bone cement component (B) above the agitaton disc (6a) and in that between said vessel bottom (4) and said open bottom end of said tubular rod (6b) there is a gap (23) forming a passage to percolate an air/liquid mixture upwardly through holes (6h) in the agitator disc (6a) to cause a pre-mixing of liquid component (A) with the powder component (B).

2. The system of Claim 1, **characterised** in that said means (1) for introducing said liquid (A) into said vessel (2) is comprised of a container (9) having an interior for containing said liquid (A), one end of said container being insertable into said open top end of said tubular rod (6b)

3. The system of Claim 1, **characterised** in that said means (1) for introducing said liquid (A) into said vessel (2) is comprised of a container (9) having an interior for containing said liquid (A) one end of said container being insertable into a funnel (50), said funnel (50) having an open neck that is removably inserted into said open end of said tubular rod (6b).

4. The system of Claim 2 wherein said container is comprised of a collapsible plastic bag.

5. A method for mixing a liquid and a powder bone cement component (A, B) in a mixing vessel (2) maintained under vacuum for the preparation of said bone cement the method comprising the steps of providing a mixing vessel (2) which
said vessel is defined by a cylindrical cylinder having an open interior (2a) with a spout (5) attached to one end of said cylinder, and having an axially displaceable bottom (4);
providing a mixing agitator (6) and inserting it within said spout so as to communicate with said vessel interior (2a), and such that it is free be slided vertically up and down while maintaining a seal in the spout (5) said agitator (6) comprised of a tubular rod (6b) having an agitator disk (6a) fixed on one end thereof, said other end being open and defining a mouth, said mouth being located axially above said spout of said vessel;
providing a tightening means introducing said liquid bone cement component (A) into the vessel (2) so as to seal said vessel (2a) from said atmosphere before and after said liquid component (A) is introduced into said vessel (2a);
removing said tightening means during introduction of said liquid component (A) into said interior of said vessel (2a) near said vessel bottom (4) under the influence of partial vacuum;
re-inserting said sealing means within said tubular rod, thereby sealing said vessel (2a) from said atmosphere;
axially displacing said agitator (6) so as to mix said liquid and powder components (A, B) under vacuum, **characterised** in that said mixing vessel (2) being provided with a pre-determined amount of said powder component of said cement and in arranging between said vessel bottom and said open second end of said tubular rod (6b) a gap (23) behaving like a passage for percolating an air/liquid mixture upwardly through holes (6h) in the agitator disc (6a) to cause liquid component (A) to premix with the powder component (B).

## Patentansprüche

1. System zum Mischen eines flüssigen und eines pulverigen Bestandteils (A, B) zur Herstellung eines Knochenzements unter Vakuum, um ein Entweichen schädlicher Emissionen zu verhindern, sobald der flüssige und der pulverige Bestandteil (A, B) gemischt werden, umfassend:
ein Mischgefäß (2), das von einer Außenwand (3) definiert wird, mit einem oberen Ende, einem unteren Ende und einem Inneren, wobei das obere Ende mit einem abdichtbaren Ausguß (5) gebildet ist und das untere Ende mit einem axial verschiebbaren Boden (4) gebildet ist;
ein Mittel (1) zum Einleiten des flüssigen Bestandteils (A) mittels eines in dem Gefäß herrschenden Vakuums durch den abdichtbaren Ausguß (5) in das Innere des Mischgefäßes (2);
einen in dem Gefäßinneren (2a) aufgenommenen Rührer (6), wobei der Rührer (6) so montiert ist, daß er frei ist, unter Aufrechterhaltung einer Abdichtung in dem Ausguß (5) zum Mischen der Bestandteile (A) und (B) vertikal aufwärts und abwärts zu gleiten, und aus einem röhrenförmigen Stab (6b), der ein offenes oberes Ende, welches eine Öffnung definiert, aufweist und nach oben durch den Ausguß (5) aus dem Inneren herausragt und mit der Atmosphäre in Verbindung steht, und einer Rührerscheibe (6a) besteht, die an einem offenen unteren Ende des röhrenförmigen Stabs in dem Gefäßinneren an dem röhrenförmigen Stab (6b) befestigt ist;
einen entfembaren Abdichtungsstab (19), der in dem röhrenförmigen Stab (6b) angeordnet ist, um vor und nach dem Einleiten des flüssigen Bestandteils (A) in das Mischgefäß (2) das offene untere Stabende gegenüber einer Verbindung mit der Atmosphäre abzudichten, wobei der Abdichtungsstab (19) unmittelbar vor dem Einleiten der flüssigen Knochenzementverbindung (A) in das Mischgefäß (2) aus dem röhrenförmigen Stab (6b) entfernt wird und wieder in diesen eingesetzt wird, nachdem die Flüssigkeit (A) in das Mischgefäß eingeleitet worden ist;
dadurch gekennzeichnet, daß das Gefäß über der Rührerscheibe (6a) mit dem pulverigen Knochenzementbestandteil (B) vorgefüllt ist und daß zwischen dem Gefäßboden (4) und dem offenen unteren Ende des röhrenförmigen Stabs (6b) ein Spalt (23) vorhanden ist, der einen Durchgang bildet, um ein Luft/Flüssigkeitsgemisch nach oben durch Löcher (6h) in der Rührerscheibe (6a) durchsickern zu lassen, um ein Vormischen des flüssigen Bestandteils A mit dem pulverigen Bestandteil B zu bewirken.

2. System nach Anspruch 1, dadurch gekennzeichnet, daß das Mittel (1) zum Einleiten der Flüssigkeit (A) in das Gefäß (2) aus einem Behälter (9) mit einem Inneren zur Aufnahme der Flüssigkeit (A) besteht, wobei ein Ende des Behälters in das offene.obere Ende des röhrenförmigen Stabs (6b) einsetzbar ist.

3. System nach Anspruch 1, dadurch gekennzeichnet, daß das Mittel (1) zum Einleiten der Flüssigkeit (A) in das Gefäß (2) aus einem Behälter (9) mit einem Inneren zur Aufnahme der Flüssigkeit (A) besteht, wobei ein Ende des Behälters in einen Trichter (50) einführbar ist, wobei dieser Trichter (50) einen offenen Hals aufweist, der herausnehmbar in das offene Ende des röhrenförmigen Stabs (6b) eingesetzt wird.

4. System nach Anspruch 2, bei dem der Behälter aus einem faltbaren Kunststoffbeutel besteht.

5. Verfahren zum Mischen eines flüssigen und eines pulverigen Knochenzementbestandteils (A, B) in einem unter Vakuum gehaltenen Mischgefäß (2) zur Herstellung des Knochenzements, wobei das Verfahren die folgenden Schritte umfaßt:
Bereitstellen eines Mischgefäßes (2), wobei dieses Gefäß von einem runden Zylinder definiert wird, der ein offenes Inneres (2a) mit einem Ausguß (5), der an einem Ende des Zylinders angebracht ist, und einen axial verschiebbaren Boden (4) aufweist;
Bereitstellen eines Mischrührers (6) und Einführen desselben in den Ausguß, so daß er mit dem Inneren (2a) des Gefäßes in Verbindung steht und derart, daß er frei ist, unter Aufrechterhaltung einer Abdichtung in dem Ausguß 5 vertikal aufwärts und abwärts zu gleiten, wobei dieser Rührer (6) aus einem röhrenförmigen Stab (6b) besteht, an dessen einem Ende eine Rührerscheibe (6a) befestigt ist, wobei das andere Ende offen ist und eine Öffnung definiert, wobei sich diese Öffnung axial über dem Ausguß des Gefäßes befindet;
Bereitstellen eines Abdichtungsmittels, das den flüssigen Knochenzementbestandteil (A) in das Gefäß (2) einleitet, um das Gefäß (2a) vor und nach dem Einleiten des flüssigen Bestandteils (A) in das Gefäß (2a) gegenüber der Atmosphäre abzudichten;
Entfernen des Abdichtungsmittels während des Einleitens des flüssigen Bestandteils (A) in das Innere des Gefäßes (2a) nahe dem Gefäßboden (4) unter dem Einfluß eines Unterdrucks;
Wiedereinsetzen des Abdichtungsmittels in den röhrenförmigen Stab, wodurch das Gefäß (2a) gegenüber der Atmosphäre abgedichtet wird;
axiales Verschieben des Rührers (6), um die flüssigen und pulverigen Bestandteile (A, B) unter Vakuum zu mischen,
dadurch gekennzeichnet, daß das Mischgefäß (2) mit einer vorbestimmten Menge des pulverigen Zementbestandteils versehen ist und daß zwischen dem Gefäßboden und dem offenen zweiten Ende des röhrenförmigen Stabs (6b) ein Spalt (23) vorgesehen ist, der als Durchgang für das Durchsickern eines Luft/Flüssigkeitsgemisches nach oben durch Löcher (6h) in der Rührerscheibe (6a) dient, um eine Vormischung des flüssigen Bestandteils (A) mit dem pulverigen Bestandteil (B) zu bewirken.

## Revendications

1. Système pour mélanger un composant liquide et un composant en poudre (A, B) pour la préparation d'un ciment osseux sous vide afin d'empêcher des émissions nuisibles de s'échapper une fois que lesdits composants liquide et en poudre (A, B) sont mélangés, comprenant :
un récipient de mélange (2) défini par une paroi extérieure (3) ayant une extrémité de dessus, une extrémité de fond et un intérieur, ladite extrémité de dessus étant formée par un goulot (5) pouvant être scellé, ladite extrémité de fond étant formée par un fond (4) pouvant être déplacé de façon axiale ;
des moyens (1) pour introduire à l'aide du vide présent dans le récipient, ledit composant liquide (A) dans ledit intérieur dudit récipient de mélange (2) par l'intermédiaire dudit goulot (5) pouvant être scellé ;
un agitateur (6) reçu à l'intérieur dudit intérieur de récipient (2a), ledit agitateur (6) étant monté de sorte qu'il est libre de coulisser verticalement vers le haut et vers le bas pour mélanger les composants (A) et (B) tout en maintenant un joint dans le goulot (5), et étant constitué d'une tige tubulaire (6b) ayant une extrémité supérieure ouverte définissant une embouchure s'étendant vers le haut en dehors dudit intérieur par l'intermédiaire dudit goulot (5) et en communication avec l'atmosphère et un disque d'agitateur (6a) fixé à ladite tige tubulaire (6b) au niveau d'une extrémité de fond ouverte de ladite tige tubulaire à l'intérieur de l'intérieur du récipient ;
une tige de renforcement amovible (19) disposée à l'intérieur de ladite tige tubulaire (6b) pour sceller ladite extrémité de tige de fond ouverte contre toute communication avec l'atmosphère avant et après l'introduction dudit composant liquide (A) dans ledit récipient de mélange (2), ladite tige de renforcement (19) étant retirée de ladite tige tubulaire (6b) immédiatement avant que ledit composé liquide (A) du ciment osseux soit introduit dans ledit récipient de mélange (2), et étant réinsérée en son sein après que ledit liquide (A) a été introduit à l'intérieur dudit récipient de mélange, caractérisé en ce que ledit récipient est rempli à l'avance par ledit composant en poudre (B) du ciment osseux au-dessus du disque d'agitateur (6a), et en ce que, entre ledit fond de récipient (4) et ladite extrémité de fond ouverte de ladite tige tubulaire (6b), il y a un espacement (23) formant un passage pour laisser passer un mélange air/liquide vers le haut à travers des trous (6h) dans le disque d'agitateur (6a) pour provoquer un mélange préalable du composant liquide (A) avec le composant en poudre (B).

2. Système selon la revendication 1, caractérisé en ce que lesdits moyens (1) pour introduire ledit liquide (A) dans ledit récipient (2) sont constitués par un conteneur (9) ayant un intérieur pour contenir ledit liquide (A), une extrémité dudit conteneur pouvant être insérée dans ladite extrémité de dessus ouverte de ladite tige tubulaire (6b).

3. Système selon la revendication 1, caractérisé en ce que lesdits moyens (1) pour introduire ledit liquide (A) dans ledit récipient (2) sont constitués par un conteneur (9) ayant un intérieur pour contenir ledit liquide (A), une extrémité dudit conteneur pouvant être insérée dans un entonnoir (50), ledit entonnoir (50) ayant un col ouvert qui est inséré de manière amovible dans ladite extrémité ouverte de ladite tige tubulaire (6b).

4. Système selon la revendication 2, dans lequel ledit conteneur est constitué par un sac de plastique pliable.

5. Procédé de mélange d'un composant liquide et d'un composant en poudre (A, B) de ciment osseux dans un récipient de mélange (2) maintenu sous vide pour la préparation dudit ciment osseux, le procédé comprenant les étapes de présence d'un récipient de mélange (2), dans lequel ledit récipient est défini par un cylindre cylindrique ayant un intérieur ouvert (2a), un goulot (5) étant fixé à une extrémité dudit cylindre, et ayant un fond (4) pouvant être déplacé de façon axiale ;
présence d'un agitateur de mélange (6) et insertion de ce dernier à l'intérieur dudit goulot de façon à communiquer avec ledit intérieur de récipient (2a) et de sorte qu'il est libre de coulisser verticalement vers le haut et vers le bas tout en maintenant un joint dans le goulot (5), ledit agitateur (6) étant constitué d'une tige tubulaire (6b) ayant un disque d'agitateur (6a) fixé sur une extrémité particulière de ce dernier, ladite autre extrémité étant ouverte et définissant une embouchure, ladite embouchure étant située de façon axiale au-dessus dudit goulot dudit récipient ;
présence de moyens de renforcement introduisant ledit composant liquide (A) du ciment osseux dans le récipient (2) de façon à sceller ledit récipient (2a) par rapport à ladite atmosphère avant et après l'introduction dudit composant liquide (A) dans ledit récipient (2a) ;
enlèvement desdits moyens de renforcement pendant l'introduction dudit composant liquide (A) dans l'intérieur dudit récipient (2a) près dudit fond de récipient (4) sous l'influence du vide partiel ;
réinsertion desdits moyens d'étanchéité à l'intérieur de ladite tige tubulaire, scellant de ce fait ledit récipient (2a) par rapport à ladite atmosphère ;
déplacement axial dudit agitateur (6) de façon à mélanger lesdits composants liquide et en poudre (A, B) sous vide, caractérisé en ce que ledit récipient de mélange (2) est muni d'une quantité prédéterminée dudit composant en poudre dudit ciment et en agençant entre ledit fond de récipient et ladite seconde extrémité ouverte de ladite tige tubulaire (6b) un espacement (23) se comportant comme un passage pour laisser passer un mélange air/liquide vers le haut à travers les trous (6h) dans le disque d'agitateur (6a) pour amener le composant liquide (A) à se mélanger à l'avance avec le composant en poudre (B).
